# EUROPEAN PATENT SPECIFICATION

(11) **EP 4 329 612 B1**
(45) Date of publication and mention of the grant of the patent: **11.06.2025**
(21) Application number: 22725440.6
(22) Date of filing: 25.04.2022
(51) Int. Cl.: A61B 5/257

(54) **MEDICAL ELECTRODE**
MEDIZINISCHE ELEKTRODE
ÉLECTRODE MÉDICALE

(30) Priority: 27.04.2021 DK PA202170191
(43) Date of publication of application: 06.03.2024
(73) Proprietor: Ambu A/S, 2750 Ballerup (DK)
(72) Inventor: KASTELIJN, Line, Leach, 2750 Ballerup (DK); OLESEN, Birthe, Bjerregaard, 2750 Ballerup (DK)
(74) Representative: Guardian IP Consulting I/S
(86) International application number: PCT/EP2022/060893
(87) International publication number: WO 2022/229099

(56) References cited:
- EP-A1- 2 050 480
- WO-A1-2017/039518
- WO-A2-01/17423
- US-A- 4 393 584
- US-A1- 2005 131 465
- US-A1- 2006 135 896
- US-A1- 2008 081 978

## Description

The present disclosure relates to a medical electrode including a disc having a top side and a bottom side, an electric connector being adapted to be electrically connected to monitoring equipment, a contact medium being arranged at the bottom side of the disc and being electrically connected to the electric connector, a layer of pressure-sensitive adhesive being applied to at least a part of the bottom side of the disc, and a protective liner being removably adhered to the bottom side of the disc by means of the pressure-sensitive adhesive.

Such surface electrodes are used for establishing electrical contact between the skin of humans or animals and electrical measuring equipment for measuring or detecting bioelectrical signals caused by physiological processes, such as the heart function. Plotting of curves indicative of this function is called electrocardiography, abbreviated as ECG, and electrodes of this type are therefore often called ECG electrodes. Surface electrodes are also used for detecting neurological signals, e.g. brains signals (electroencephalograph; EEG signals) or neurological signals related to muscle activity (electromyograph, EMG signals). The present disclosure is applicable to any kind of surface electrodes.

Electrocardiography is typically performed for diagnostic purpose or for monitoring purpose.

In diagnostics, the procedure typically comprises recording a 12-lead ECG where ten electrodes are placed on the patient's limbs and on the surface of the chest. The overall magnitude of the heart's electrical potential is then measured from twelve different angles ("leads") and is recorded over a period of time, e.g. for 10 seconds or more, e.g. up to a few hours, i.e. short term applications. In this way, the overall magnitude and direction of the heart's electrical depolarization is captured at each moment throughout the cardiac cycle. Therefore, it is important to position each biomedical electrode accurately at the patient in order to obtain optimal quality ECG diagrams for each of these 12 leads.

In monitoring, including Holter, event or telemetry applications, the patient is often monitored either at the hospital or at home over a longer period of time, e.g. from about 24 hours, such as up to one week, for several weeks or sometimes for several months. In such long term applications the medical electrodes are applied for a longer time so such monitoring applications are typically called long term applications. 3 lead or 5 lead ECG are mostly used in long term applications. In hospitals, cardiac monitoring using electrocardiography is common or necessary in many units, which includes intensive care units (ICU).

US 4,524,775 (Medicotest A/S) discloses a medical electrode for contacting the skin, comprising a foam plastic disc which is formed with an aperture, and the part of the disc surrounding the aperture is covered by a cover foil on a top side thereof. A transducer strip is placed below the foil in the aperture and is connected to the stripped end of a plastic insulated lead, whose end portion is covered by the cover foil. A medical electrode having an offset connector in the form of a snap lock is also disclosed.

Medical electrodes are generally provided with a protective liner in order to protect the contact medium and the pressure-sensitive adhesive applied to the bottom side of the electrode before application of the electrode to the skin of humans or animals. In order to apply the electrode to the skin, the clinician must remove the protective liner from the electrode. The protective liner may either have the form of a large sheet with several medical electrodes attached thereto or it may have the form of a single liner used for a single medical electrode. The single liner typically has a flap that enables the clinician to grab and peel off the protective liner before applying the medical electrode to the skin of the patient. The protective liner may comprise a release layer, e.g. by being a siliconized film, to ensure easy release or delamination, and a corresponding low pull force needed for removal of the liner from the pressure-sensitive adhesive on the skin side of the medical electrode.

GB2341104 B2 describes the production of a medical electrode where a plurality of medical electrodes are arranged on a common protective liner. These electrodes comprise a disc having a top layer of a polymer film, e.g. a polyester (PET) film, onto which a foam layer, e.g. of polyethylene (PE) is applied towards the patient. An acrylic adhesive is applied to the skin side as a pressure sensitive adhesive (also called skin adhesive). The transducer element is printed onto the lower side of the top layer.

However, it may pose a challenge that, in order to remove the protective liner from the electrode, the clinician is required to use both hands. The first hand is used to hold the electrode while the other hand grips and peels off the protective liner from the electrode.

According to normal procedure in a 12 lead ECG recording, the clinician would first examine the patient to search for the correct position to apply the electrode to the patient. After finding the right position, the clinician has to remove the protective liner from the electrode which requires the use of both hands. Therefore, the clinician must remove the finger and take her/his eyes off the found application spot. Subsequently, the clinician may with the eyes find the correct position again for the application of the electrode to the skin. The clinician then applies the electrode to the patient at the previously selected position. However, the position of actual application could potentially be incorrect, as the clinician has looked away from the application spot when removing the protective liner. As it is understood, this procedure of application may lead to wrong positioning of the medical electrode.

In long term applications, which includes monitoring, Holter ECG, the patient is equipped with 4-5 biomedical electrodes, or sometimes more, which are worn for a prolonged period of time, e.g. from a few hours and up to many days, which in extreme situations could be weeks or months. In 3 lead or 5 lead ECG the accuracy when positioning each of the bioelectrodes is of less importance. In long term applications the biomedical electrode has to remain attached to the patent for the entire wear time, which could be for 3, 5 or even 7 days or more, e.g. up to 10 days. In such situations it is important that the clinician does not touch the adhesive side of the electrode as it may reduce the adhesive effect, and in the end negatively affect the wear time of the electrode, ( i.e. the time where the biomedical electrode remains on the body and provides a signal ) because the biomedical electrode may start to lift at the edges.

Furthermore, the clinician may often end up touching the adhesive on the skin side of the electrode with at least one finger when removing the protective liner. This can reduce the adhesive effect in that area and/or cause transfer of microorganisms or pathogens from the clinicians finger or glove to the patient's skin.

In addition, if the clinician is wearing gloves, the gloves may stick to the adhesive layer on the electrode which causes problems with handling and/or applying the electrode.

EP 2 050480 A1 describes an electrode pad for low frequency electrotherapy having three terminal studs aligned at the center axis of the upper side of the pad. The wires comprise a connector arrangement with three connectors arranged in a common strap or band, which can be removably attached to the terminal studs on the pad. When attached to the pad, the connector arrangement forms a finger grip between the terminal studs and a user can insert his or her fingers to ease handling of the electrode when attaching the pad t the patient's body. However, this connector arrangement will not be suitable for holding the electrode when removing (delaminating) the protective liner from the adhesive layer on the skin side of the electrode pad. The force needed for delaminating the protective liner from the adhesive layer on the skin side will be high if pulling the electrode at the center (where the terminal studs are arranged) because the user must overcome the adhesion force (between the skin adhesive and the protective liner) over the entire pad's area before the delamination occurs. So, in an attempt to remove the protective liner of this electrode pad by using the connector arrangement's finger grips for pulling the force pulling in the connector arrangement, it is more likely that the connector arrangement detaches from the connector studs or even is damaged. Also, this solution would not work on biomedical electrodes having only one terminal stud because the connector cannot form the finger grip.

US 2008/081978 A1 discloses a medical apparatus including a substrate member and an applicator member. The substrate member includes a medical device and an adhesive layer for adhering to body tissue. The applicator member is releasably attached to the substrate member and is adapted for grasping by a clinician to facilitate application of the substrate member to body tissue. In an embodiment of the medical apparatus, the applicator member incorporates a loop or key ring tab which defines an opening for receiving a clinician's finger.

WO 2017/039518 A1 discloses an electrocardiography electrode device or patch adapted for attaching to the skin of a patient, and adapted for positioning in the axilla of said patient, said device comprising an adhesive patch and electrodes positioned on said adhesive patch. In an embodiment, an additional layer forms a pocket capable of holding a communication device in a snug fit.

The object of the present disclosure is to provide a medical electrode being easier to apply correctly on the skin of a patient.

In view of this object, a finger grip is arranged at the top side of the disc, and the finger grip forms a finger insert opening into which a fingertip may be inserted and thereby hold the medical electrode during handling. The finger grip has the form of a pouch forming the finger insert opening.

In this way, a clinician may by means of one single hand grip and hold the medical electrode, remove the protective liner from the medical electrode and apply the medical electrode to the skin. In order to do this, a first finger of the single hand, such as the index finger, is inserted into the insert opening of the finger grip of the electrode, and a second finger of the single hand, such as the thumb, is used to peel off and remove the protective liner from the medical electrode by pressing on an edge part of the protective liner. In the case of a large protective sheet with several medical electrodes attached thereto, this may be done by pressing on a part of the protective sheet surrounding the medical electrode to be applied, for instance by holding the large protective sheet towards a table with fingers from the same hand where one finger is engaged in the finger grip. While still holding the medical electrode by means of the first finger inserted into the finger grip, the electrode may be placed correctly on the skin of the patient. This procedure allows the clinician to maintain a finger of her/his other hand on the previously found right spot of the skin while removing the protective liner. This allows the clinician to place the electrode correctly.

Therefore, advantageously, because the medical electrode according to the present disclosure is one hand applicable to the skin, correct application and positioning of the electrode on the skin is greatly facilitated.

Furthermore, it may be advantageous that, by means of the medical electrode according to the present disclosure, there may be a reduced risk of touching the adhesive layer or the contact medium which are clean areas, because the clinician may by means of a first hand use the finger grip to apply the electrode to the patient while identifying the correct application spot with the other hand. The fingers of the hand holding the electrode may not easily come into contact with said clean areas at the bottom side of the electrode, because that hand is fixed at the top side of the electrode by means of the finger grip.

Due to the finger grip having the form of a pouch forming the finger insert opening, a secure grip of the medical electrode may be obtained when a finger is inserted into the pouch.

In an embodiment, the finger grip has the form of a strap having opposed ends being attached to the medical electrode. The opposed ends of the strap may be glued, welded or hot melted to an upper surface of the medical electrode.

In an embodiment simple to manufacture, the finger grip has the form of a plastic loop that is attached to an upper surface of the medical electrode. For instance, the plastic loop may be glued, welded or hot melted to the upper surface of the medical electrode.

In yet a further variant, the finger strap may simply be a thin strap or tape attached to the upper side of the medical electrode. The thin strap or tape may further be arranged to lie flat across and/or against the upper side of the biomedical electrode.

In an embodiment, the finger grip is tapered in such a way that an internal cross-sectional area is reduced gradually with increasing distance from the finger insert opening. Thereby, the finger grip may better fit the form of a fingertip and therefore the medical electrode may be more securely supported by the fingertip. Furthermore, by being tapered, the finger grip may be adapted to better fit different finger sizes.

In an embodiment, the protective liner is a separate liner for said medical electrode, and the protective liner is provided with at least one grip flap protruding from an edge of the disc. Thereby, the application of the medical electrode by means of a single hand may be further facilitated in that the at least one grip flap protruding from an edge of the disc may facilitate the removal of the protective liner from the medical electrode by means of another finger on the same hand than the finger used to hold the electrode by being inserted into the finger grip.

Alternatively, a plurality of medical electrodes may be provided on a single common liner. The number of biomedical electrodes on a single, preferably flat, liner may depend on the intended application. In non-limiting examples the number of biomedical electrodes on a single liner may be 2,3,4,5,6,7,8,9, 10, 11, 12 or more with 3, 4 or 5 or 10 biomedical electrodes being preferred.

In an embodiment, the finger grip defines an insert direction for the insertion of a fingertip into the finger grip, and the at least one grip flap protrudes in the insert direction or a transverse direction thereof. Thereby, the application of the medical electrode by means of a single hand may be further facilitated in that the at least one grip flap protruding in the insert direction or a transverse direction thereof may easily be reached by means of another finger than the finger used to hold the electrode by being inserted into the finger grip. For instance, the index finger of one hand may be inserted into the finger grip in the insert direction, in which case the at least one grip flap protruding in the insert direction or a transverse direction thereof may easily be reached by another finger on the same hand, e.g. the thumb of the same hand or for instance by the middle finger of the same hand.

In an embodiment, the at least one grip flap has an uneven surface, possibly provided with a number of surface protrusions, such as knobs. Thereby, the removal of the protective liner from the medical electrode may further be facilitated in that an even more secure grip may be provided for the finger or fingers used to press on the at least one grip flap.

In an embodiment, the protective liner protrudes from the edge of the disc along a circumference of the disc by at least a minimum protrusion distance, the at least one grip flap of the protective liner protrudes from the edge of the disc by a distance of at least 5 times, and preferably at least 8 times, the minimum protrusion distance. Thereby, a suitable area for the at least one grip flap of the protective liner may be provided without using much material for the protective liner along the entire circumference of the disc. Advantageously, the protective liner may protrude from the edge of the disc along a circumference of the disc by at least a minimum protrusion distance in order to maintain clean the edge of the layer of pressure-sensitive adhesive. However, this minimum protrusion distance may be too little for a finger to press on in order to remove the protective liner from the electrode.

In an embodiment, the electric connector is offset in relation to a central part of the disc of the medical electrode, and the electric connector, the central part of the electrode and the finger grip are aligned along a centre line of the medical electrode extending in the insert direction defined by the finger grip. In this embodiment, when peeling off the protective liner, it could appear natural to grab the offset connector, if a grip flap would be provided below the offset connector, as it is normal in prior art medical electrodes. However, if doing so, there would be a risk of damage to the electrical connection between the contact medium and the electric connector. This connection is critical for obtaining a good signal. Therefore, according to this embodiment, it may be a particular advantage that the at least one grip flap protrudes in the insert direction or a transverse direction thereof. Thereby, it may be avoided that a user inadvertently grips the offset connector instead of the at least one grip flap on the collar.

In an embodiment, an aperture extends at a central part of the disc from the top side to the bottom side and thereby forms a contact medium chamber in which the contact medium is arranged, and the finger grip is arranged at the top side of the surrounding part of the disc. Thereby, the position of the finger grip may induce a user to press the medical electrode against the patient's skin at the surrounding part of the disc rather than at the central part of the disc on the contact medium chamber in which the contact medium is arranged. Thereby, it may advantageously be avoided that contact medium, such as gel, may splash out from the contact medium chamber. If the gel is pressed out between the skin and the adhesive layer, then there may be adhesion problems. Also, if gel is pressed out of the contact medium chamber, there may be insufficient gel left in the chamber to establish a proper electrical connection between the skin and the transducer. Usually, the clinician presses the medical electrode against the patient's skin in order to ensure that the electrode is firmly attached to the skin prior to taking ECG's.

The finger strap may work equally well on biomedical surface electrodes where a conductive adhesive is applied to the skin surface of the biomedical electrode.

In a structurally particularly advantageous embodiment simple to manufacture, a plastic foil having a top side and a bottom side is arranged on the top side of the disc, thereby covering the aperture and at least a part of the surrounding disc, the plastic foil being welded to the disc along a weld line surrounding the aperture, and the finger grip is formed as a part of the plastic foil or is attached to the plastic foil.

In an embodiment, the finger grip is detachably arranged on the medical electrode, e.g. by applying perforated tear lines. This may be advantageous, for instance if the patient is wearing clothes when the electrode is applied to the skin. Thereby, any inconvenience for the patient may be avoided.

In an embodiment, the finger grip is attached to the medical electrode at a distance from the electric connector. Thereby, because there may be no interference between the finger grip and the electric connector, you may, by using a single hand, remove the protective liner and/or attach the biomedical electrode to the patients body by using the finger strap without having any leads attached thereto. This may be advantageous in that the lead or leads may be connected to the electrode or electrodes after applying the electrode or electrodes to the skin, thereby facilitating the application in that the leads may not get in the way during application of the electrode or electrodes.

In an embodiment, the finger grip is arranged eccentrically in relation to a central part of the disc of the medical electrode or the finger grip is arranged at an edge of the disc of the medical electrode. Thereby, the point where the clinician will be pulling in the electrode by means of the finger grip may be close to the edge of the disc. Thus, the force needed to delaminate the protective liner from the adhesive layer on the bottom side of the disc may be minimized and thereby the removal of the protective liner may be facilitated.

The disclosure will now be explained in more detail below by means of examples of embodiments with reference to the very schematic drawing, in which
Figs. 1 to 3 illustrate different stages of placement of a prior art medical electrode on the skin of a patient,
Figs. 4 to 10 illustrate different stages of placement of a medical electrode according to the present disclosure on the skin of a patient,
Figs. 11 and 12 illustrate the forming of a first finger grip for a medical electrode according to the present disclosure,
Figs. 13 and 14 illustrate the forming of a second finger grip for a medical electrode according to the present disclosure,
Figs. 15 and 16 illustrate the forming of a third finger grip for a medical electrode according to the present disclosure,
Fig. 17 is a perspective view of a medical electrode according to the present disclosure with the first finger grip and a first protective liner, whereby the protective liner has been removed,
Fig. 18 is a perspective view of the medical electrode of Fig. 17, whereby the protective liner is attached to the medical electrode,
Fig. 19 is a perspective view of a medical electrode according to the present disclosure with the first finger grip and a second protective liner, whereby the protective liner has been removed,
Fig. 20 is a perspective view of a medical electrode according to the present disclosure with the first finger grip and a third protective liner, whereby the protective liner has been removed,
Fig. 21 is a perspective view of a medical electrode according to the present disclosure with the second finger grip and a fourth protective liner, whereby the protective liner has been removed,
Fig. 22 is a perspective view of a medical electrode according to the present disclosure with the third finger grip and a fifth protective liner, whereby the protective liner has been removed,
Fig. 23 is a longitudinal section through the medical electrode of Fig. 22, without the protective liner, and
Figs. 24 and 25 illustrate an embodiment of a large sheet-like protective liner 36 carrying a plurality of medical electrodes.

In the following, generally, similar elements of different embodiments have been designated by the same reference numerals.

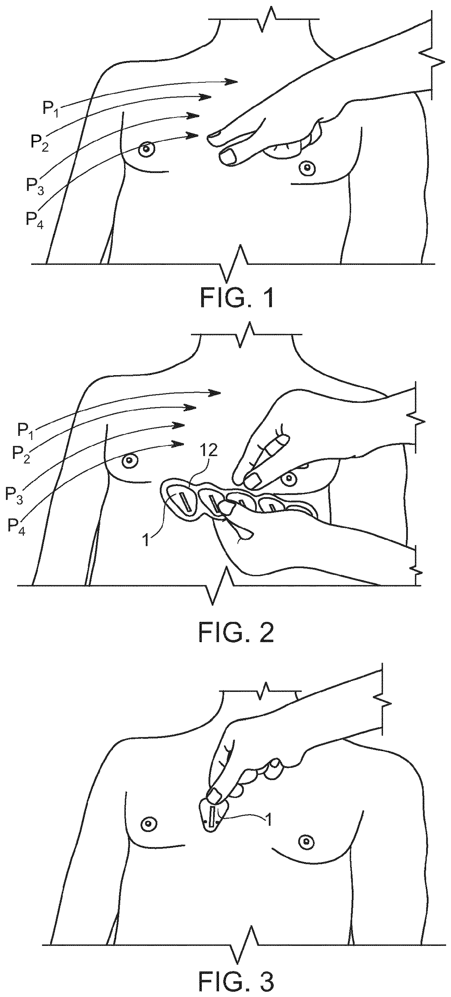

Figs. 1 to 3 illustrate how a prior art medical electrode 1 is typically placed on the skin of a patient in order to perform ECG. Depending on the setup, a number of 3-4 and up to 12 electrodes may be applied to detect ECG signals. In Fig. 1, the clinician examines the patient by means of a first hand (in this case her/his right hand) to search for the correct position P₁, P₂, P₃, P₄ to apply the electrode to the patient. In Fig. 2, the clinician uses the other hand (in this case her/his the left hand) to hold the medical electrode 1 while the other (right) hand grips and peels off a protective liner 12 from the electrode. In Fig. 3, the clinician uses her/his the right hand to place the medical electrode 1 on the previous found spot on the skin.

However, it may pose a challenge that, in order to remove the protective liner 12 from the medical electrode 1, the clinician is required to use both hands. As seen in Fig. 2, after finding the right position in Fig. 1, the clinician has to remove the protective liner 12 from the electrode which requires the use of both hands. Therefore, the clinician must remove the finger and take her/his eyes off the found application spot. Subsequently, the clinician may with the eyes find the correct position again for the application of the electrode to the skin. The clinician then applies the electrode to the patient at the previously selected position. However, the position of actual application could potentially be incorrect, as the clinician has looked away from the application spot when removing the protective liner 12. As it is understood, this procedure of application may lead to wrong positioning of the medical electrode 1.

It is noted that the present disclosure is not limited to use exclusively with ECG electrodes and electrocardiography. The suggested solution is equally applicable with all types of biomedical surface electrodes, including biomedical surface electrodes used in neurological studies, such as detecting bioelectrical brain signals, called EEG or electroencephalography, or detecting bioelectrical signals in muscle tissue, called EMG or electromyography.

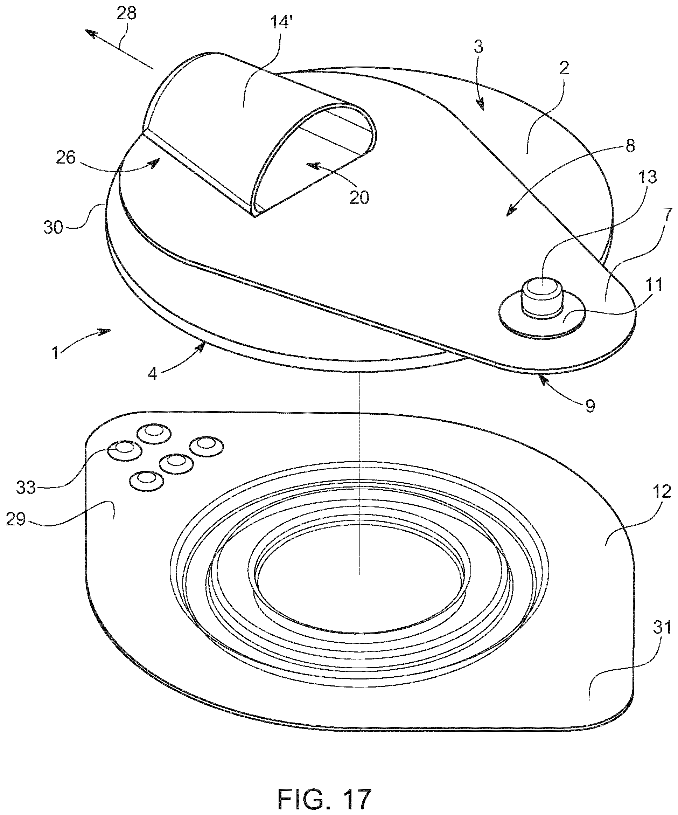

Figs. 17 to 23 illustrate different embodiments of a medical electrode 1 according to the present disclosure. The medical electrode 1 includes a disc 2, e.g. in the form of a plastic foam disc 2, having a top side 3 and a bottom side 4. The disc 2 may be more or less circular or oval or may have any other suitable form. The medical electrode 1 may typically be of the type used for establishing electrical contact between the skin of humans or animals and electrical measuring equipment, e.g. when performing electrocardiography. The electrode could for instance be a BlueSensor R available from Ambu (Registered Trademark) which is used as a non-limiting example below and shown in the figures.

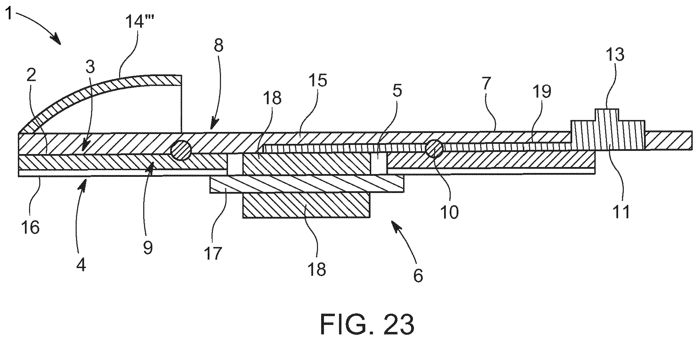

In order to establish said electrical contact, a contact medium 18 may be arranged at the bottom side 4 of the disc 2 and is electrically connected to an electric connector 11. As seen in the embodiment illustrated in Fig. 23, a preferably circular aperture 5 may extend at a central part of the disc 2 from the top side 3 to the bottom side 4 of the disc 2 and thereby forms a contact medium chamber 6. A plastic foil 7 has a top side 8 and a bottom side 9 and is arranged on the top side 3 of the disc 2, thereby covering the aperture 5 and a part of the surrounding disc 2. In the illustrated embodiment, the bottom side 9 of the plastic foil 7 is welded to the top side 3 of the disc 2 along a weld line 10 surrounding the aperture 5. However, the plastic foil 7 may be attached to the disc 2 in any suitable way, such as by gluing. In the illustrated embodiment, the plastic foil 7 extends to the edge of the disc 2, where the finger grip 14‴ is attached. However, the plastic foil 7 does not need to extend to the edge of the disc 2, whereas the finger grip 14‴ may still be attached to the plastic foil 7 and extend further in the direction of the edge of the disc 2 than the extension of the plastic foil 7. Furthermore, as seen, the above-mentioned electric connector 11 is mounted on the plastic foil 7 eccentrically in relation to the central part of the disc 2 at which the aperture 5 is arranged. In other embodiments, the electric connector 11 may be mounted centrally. As seen, in the illustrated embodiments, the plastic foil 7 has a teardrop form with the electric connector 11 arranged at a narrow end of the teardrop form. The electric connector 11 is adapted to be electrically connected to not shown monitoring equipment in a well-known way by means of a not shown cable in that the electric connector 11 has a first snap lock part 13 to which a second not shown snap lock part of said cable may connect. In the illustrated embodiments, the first snap lock part 13 is a male connector and the second not shown snap lock part is a female connector. Such female connector part may often be referred to as "cable connector". It may actually also be a crocodile clip. An alternative connector can be an "interface plug" arranged on the male stud on the electrode (or integrated instead of the male stud) and may be adapted to receive a banana plug. Alternatively, the cable or wire can be integrated into the electrode, e.g. as in Ambu^{®} BlueSensor QR.

As further seen in Fig. 23, a transducer strip 15 is disposed in the contact medium chamber 6 and is electrically connected to the electric connector 11 by means of an extension 19 of the transducer strip 15 or possibly by means of a dedicated conductive element. The transducer strip 15 may for instance have the form of a metal strip, for instance a silver chloride coated silver strip, a carbon strip, a silver coated and/or silverchloride coated polymeric sheet layer or a sensor area printed by means of conductive ink, e.g. comprising carbon or a Ag/AgCl, containing ink on a separate strip or on the lower side of the plastic foil 7. A layer 16 of pressure-sensitive adhesive is applied to the bottom side 4 of the disc 2 for adhesion to the skin of the patient. Suitable skin adhesives are well-known, and may e.g. be acrylate based or silicone based adhesives.

A sponge 17 may be arranged across the contact medium chamber 6, e.g. by a peripheral edge of the sponge 17 being adhered to the bottom side 4 of the disc 2 by means of the layer 16 of pressure-sensitive adhesive. The necessary electrical contact between the transducer strip 15 and the skin of a person can be established by means of the contact medium 18 which has been at least partly absorbed in the sponge 17. The contact medium 18 may generally be a paste-like electrolyte. The contact medium 18 is typically a conductive (wet) gel. The conductive gel typically comprises ions, such as chloride ions ( Cl⁻) that transfer bioelectric signals from the patient's skin to the transducer strip 15. Preferably, the gel is a NaCl + KCl containing gel. The layer 16 of pressure-sensitive adhesive applied to the bottom side 4 of the disc 2 may ensure that the medical electrode 1 is securely adhered to the skin of a person during use.

As patients are often to be monitored for long periods of time, e. g. several days, it is important that the medical electrode 1 adds as little to the discomfort of the patient or carrier as possible. This is provided for to a great extent by the use of the disc 2, which is a soft and pliable material. The preferred foam plastics for the disc 2 is PVC foam; however, other materials may be used, such as polyolefines, natural or synthetic rubber, biopolymers, polyesters, polyethers, polyurethanes, polyamides, or copolymers such as ABS or mixtures thereof or laminates comprising one or more of these may further be used.

It is also important that the gel-like contact medium 18, which is generally used for establishing good electrical connection between the transducer strip 15 and the skin, can be kept intact during the entire period of operation. This means that the walls defining the contact medium chamber 6 must be so tight as to allow no considerable diffusion of the constituent components of the contact medium 18.

Although in Fig. 23, as explained above, it has been illustrated that the plastic foil 7 is welded to the top side 3 of the disc 2 along a weld line 10 surrounding the aperture 5, in reality, the weld line 10 may be broader than illustrated and may extend to the edge of the contact medium chamber 6 as it will be explained in the following. The preferred material for the plastic foil 7 which in the case of the Ambu BlueSensor is in blue colour, is polyvinylchloride (PVC); however, other materials may be used, such as polyolefines, such as polyethylene or polypropylene, natural or synthetic rubber, biopolymers, polyesters, polyethers, polyurethanes, polyamides or copolymers such as ABS or mixtures thereof or laminates comprising one or more of these may also be used.

The medical electrode 1 illustrated may generally be manufactured by placing the various parts in their proper mutual positions between a not shown flat metal substrate and a not shown metal pressing plate, and whereby the pressing plate is subsequently moved so far down towards the substrate that the part of the disc 2 aligned with the plastic foil 7 is somewhat compressed. The substrate and the pressing plate may form a capacitor inserted in a high-frequency circuit (not shown), and heat may be developed between these plates as a consequence of dielectric losses. The heat causes walls of compressed plastic foam cells to fuse partly so that the part of the disc 2 in question is fixed in the compressed shape. At the same time the plastic foil 7 is welded to the disc 2. The extension 19 of the transducer strip 15 extends through the welding formed to the electric connector 11. Other suitable manufacturing methods are, of course, also possible. The transducer strip is typically an AgCl coated silver strip, but could in principle also be printed (using e.g. conductive ink or carbon) on the lower side of the foil 7 (or an intermediate foil layer).

As already mentioned above, medical electrodes 1 are generally provided with a protective liner 12 in order to protect the contact medium 18 and the layer of pressure-sensitive adhesive 16 applied to the bottom side of the electrode before application of the electrode to the skin of humans or animals. In order to apply the electrode to the skin, the clinician must remove the protective liner from the electrode.

According to the present disclosure, the protective liner 12 is adhered to the bottom side 4 of the disc 2 by means of the pressure-sensitive adhesive 16. Furthermore, according to the present disclosure, a finger grip 14', 14", 14‴ is arranged at the top side of the disc 2, and the finger grip forms a finger insert opening 20 into which a fingertip may be inserted and thereby hold the medical electrode 1 during handling.

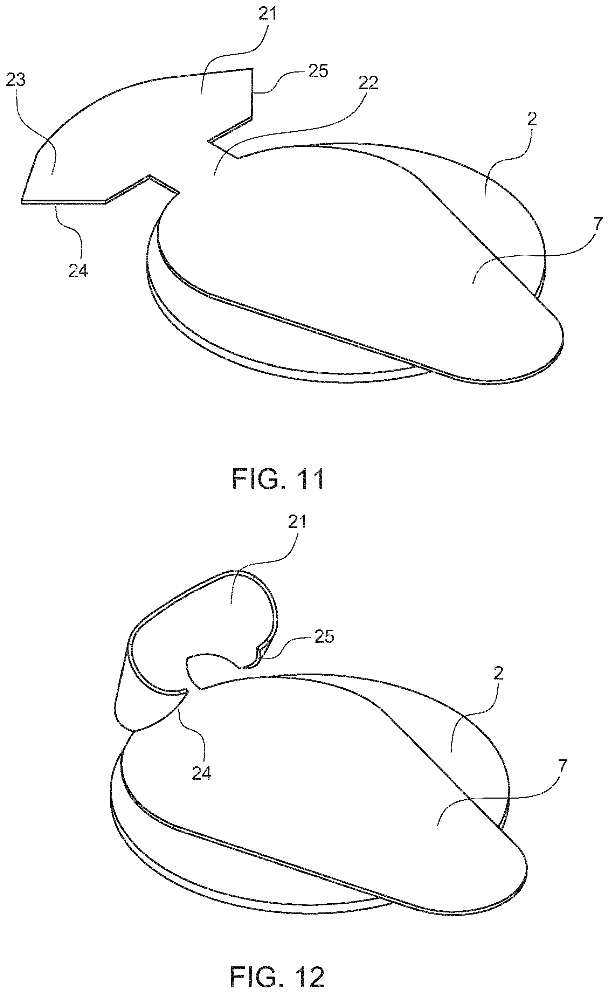

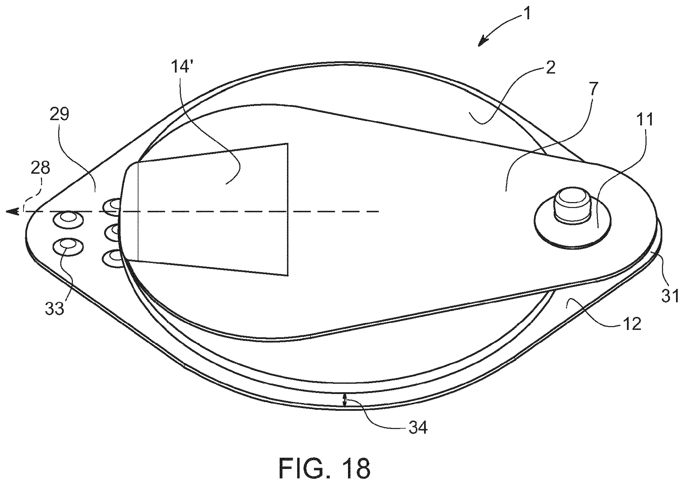

According to the embodiment of Figs. 17 and 18, a medical electrode 1 according to the present invention is provided with a first finger grip 14' (a first embodiment of the finger grip) which has been formed by suitably folding a T-formed extension 21 of the plastic foil 7 and welding or gluing it to itself as illustrated in Figs. 11 and 12. As seen, the T-formed extension 21 has been cut so that it is composed by a first part 22 and a traverse part 23. The first part 22 connects a teardrop form of the plastic foil 7 to the traverse part 23. Opposed ends 24, 25 of the traverse part 23 are welded, hot melted or glued to the top side 8 of the teardrop form of the plastic foil 7. Thereby, as seen in Fig. 17, the first finger grip 14' is formed as a pouch 26 forming the finger insert opening 20. A bottom of the pouch 26 is formed by first part 22 of the T-formed extension 21 of the plastic foil 7. Because in this embodiment, the opposed ends 24, 25 of the traverse part 23 are cut obliquely to each other and subsequently arranged parallel to each other when welded, hot melted or glued to the plastic foil 7, as seen in Fig. 17, the finger grip 14' is tapered in such a way that an internal cross-sectional area is reduced gradually with increasing distance, in direction 28 in Fig. 17, from the finger insert opening 20. Thereby, the finger grip 14' may better fit the form of a fingertip and therefore the medical electrode 1 may be more securely supported by the fingertip. Furthermore, by being tapered, the finger grip 14' may be adapted to better fit different finger sizes.

The folded finger grip 14', 14" could also be welded, glued or hot melted directly on the top of the disc 2 or the foil 7 or when welding the teardrop shaped foil onto the disc 7.

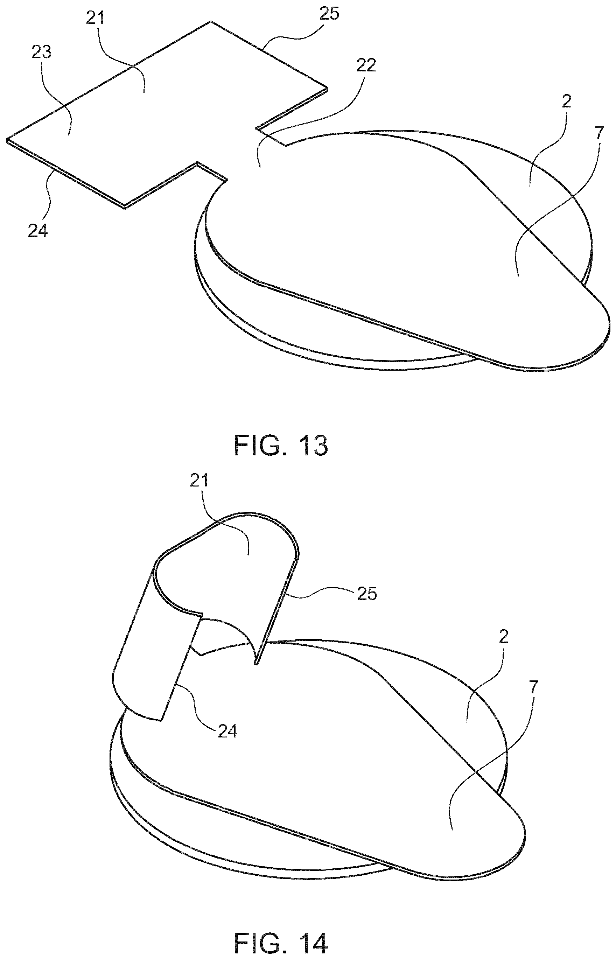

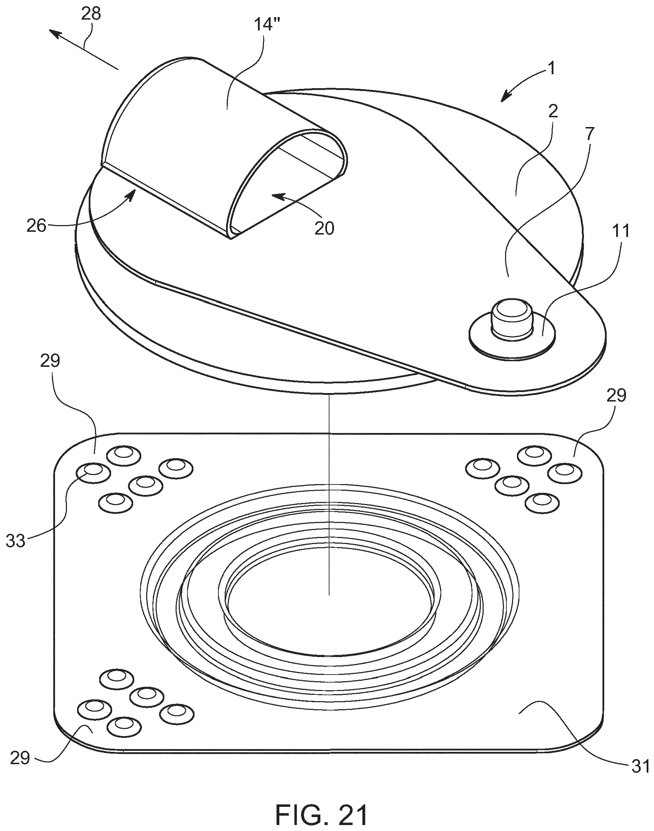

According to the embodiment of Fig. 21, a medical electrode 1 according to the present invention is provided with a second finger grip 14" (a second embodiment of the finger grip) which has been formed by suitably folding a T-formed extension 21 of the plastic foil 7 and welding, hot melting or gluing it to itself as illustrated in Figs. 13 and 14. As seen, the T-formed extension 21 has been cut so that it is composed by a first part 22 and a traverse part 23. The first part 22 connects a teardrop form of the plastic foil 7 to the traverse part 23. Opposed ends 24, 25 of the traverse part 23 are welded, hot melted or glued to the top side 8 of the teardrop form of the plastic foil 7. Thereby, as seen in Fig. 21, the second finger grip 14" is formed as a pouch 26 forming the finger insert opening 20. A bottom of the pouch 26 is formed by first part 22 of the T-formed extension 21 of the plastic foil 7. Because in this embodiment, the opposed ends 24, 25 of the traverse part 23 are cut parallel to each other and subsequently also arranged parallel to each other when welded, hot melted or glued to the plastic foil 7, as seen in Fig. 21, the finger grip 14" is not tapered as in the embodiment illustrated in Fig. 17.

As seen, in the embodiments of Figs. 17 and 21, the finger grip 14', 14" has the form of a strap or a plastic loop having opposed ends being attached to the medical electrode 1. In this case, the strap or plastic loop is formed by the traverse part 23 of the T-formed extension 21 of the plastic foil 7, and the strap or plastic loop is further at its middle connected to the plastic foil 7 by means of the first part 22 of the T-formed extension 21. The loop/cone shape of the finger grip 14', 14" shown in these figures could also be formed separately from the teardrop shaped top foil 7 and attached to the foil by welding, hot melting or gluing the cone/loop to the top surface of the electrode , e.g. to the foil 7 and/or the foam disc 2.

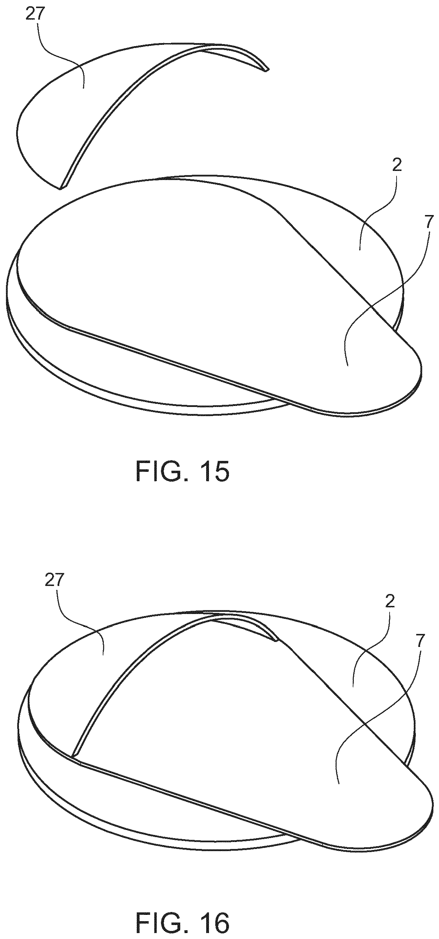

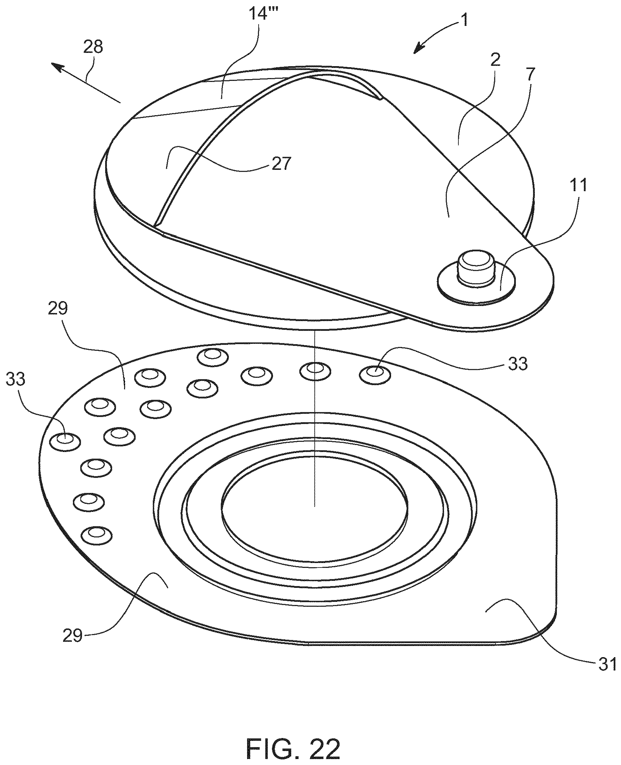

According to the embodiment of Figs. 22 and 23, a medical electrode 1 according to the present disclosure is provided with a third finger grip 14'" (a third embodiment of the finger grip) which has been formed by welding or gluing a separate piece 27 of plastic foil on top of the plastic foil 7 of the medical electrode in such a way that a pouch 26 is formed as illustrated in Figs. 15 and 16. As seen, the separate piece 27 of plastic foil has been cut so that it conforms to part of the teardrop form of the plastic foil 7 and has subsequently been welded, hot melted or glued to the top side 8 of the teardrop form of the plastic foil 7 along part of the edge of said teardrop form. Thereby, as seen in Fig. 22, the third finger grip 14‴ is formed as a pouch 26 forming the finger insert opening 20. Furthermore, the separate piece 27 of plastic foil has been cut in such a form that when it has been attached to the part of the edge of the plastic foil 7, as seen in Fig. 22, the third finger grip 14‴ is tapered in such a way that an internal cross-sectional area is reduced gradually with increasing distance from the finger insert opening 20. Thereby, the third finger grip 14‴ may better fit the form of a fingertip and therefore the medical electrode 1 may be more securely supported by the fingertip. Furthermore, by being tapered, the third finger grip 14‴ may be adapted to better fit different finger sizes. As it will be understood, according to this embodiment, the separate piece 27 of plastic foil may be made of the same or of a different material than that of the plastic foil 7 and it may be attached to the electrode in the same welding step that attaches the top foil to the foam disc.

As seen, in the embodiments illustrated in Figs. 17 to 22, the protective liner 12 is a separate liner for said medical electrode 1, and the protective liner 12 is provided with at least one grip flap 29 protruding from an edge of the disc 2.

In an embodiment, protective liner 12 is made of a plastic material having a tensile modulus being higher than 0.1 GPa and preferably higher than 1 GPa (Test method: ASTM-D-882:18). The material of protective liner 12 may be a combination film in the form of A-PET - LDPE laminate or film or other alternative stiff materials such as PE, PP, ABS, PET or laminates comprising one or more layers thereof. The protective disc-formed structure 20 may have a thickness of for instance 0.3 millimetres.

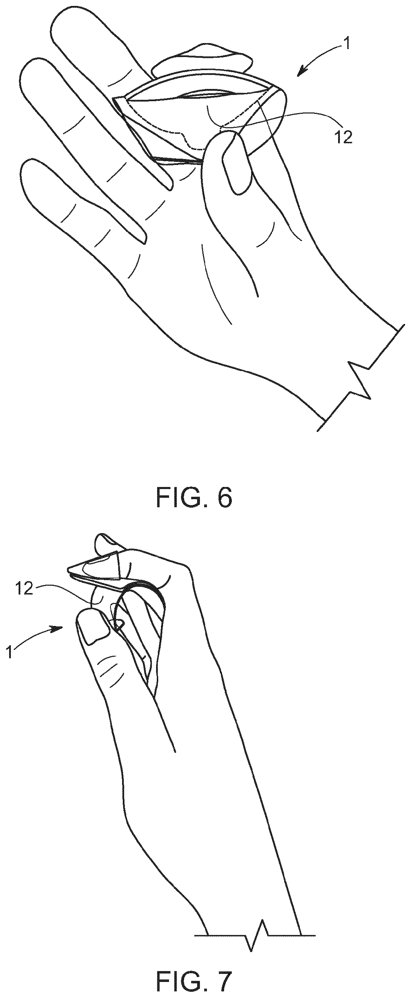

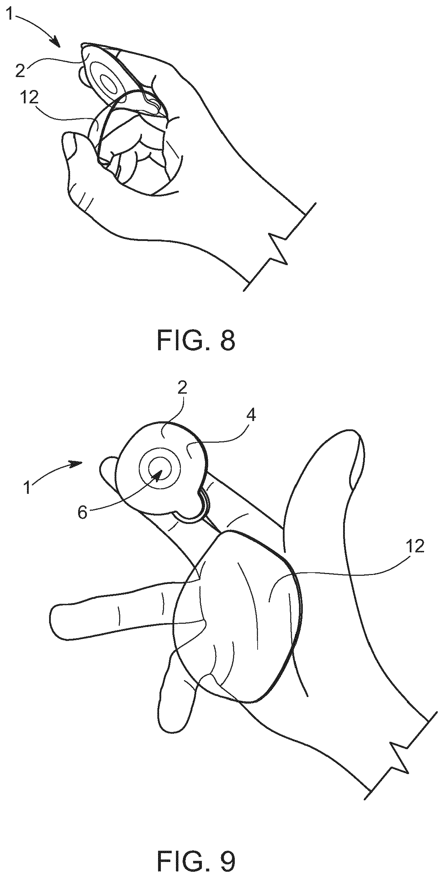

According to the embodiment of Figs. 17 and 18, the finger grip 14' defines an insert direction 28 for the insertion of a fingertip into the finger grip, and a first grip flap 29 (corresponding to a first embodiment of the protective liner 12) protrudes from an edge 30 of the disc 2 in the insert direction 28. Thereby, the application of the medical electrode 1 by means of a single hand may be further facilitated in that the grip flap 29 protruding in the insert direction 28 may easily be reached by means of another finger than the finger used to hold the electrode by being inserted into the finger grip. For instance, the index finger of one hand may be inserted into the finger grip in the insert direction, in which case the grip flap 29 protruding in the insert direction 28 may easily be reached by the thumb of the same hand or for instance by the middle finger of the same hand, as illustrated in Figs. 6 to 10. It is noted that according to this embodiment of the protective liner 12, the protective liner 12 is preferably also provided with a protective flap 31 arranged opposed to the grip flap 29 an below the electric connector 11 in order to protect the electric connector 11 and thereby also the extension 19 of transducer strip 15.

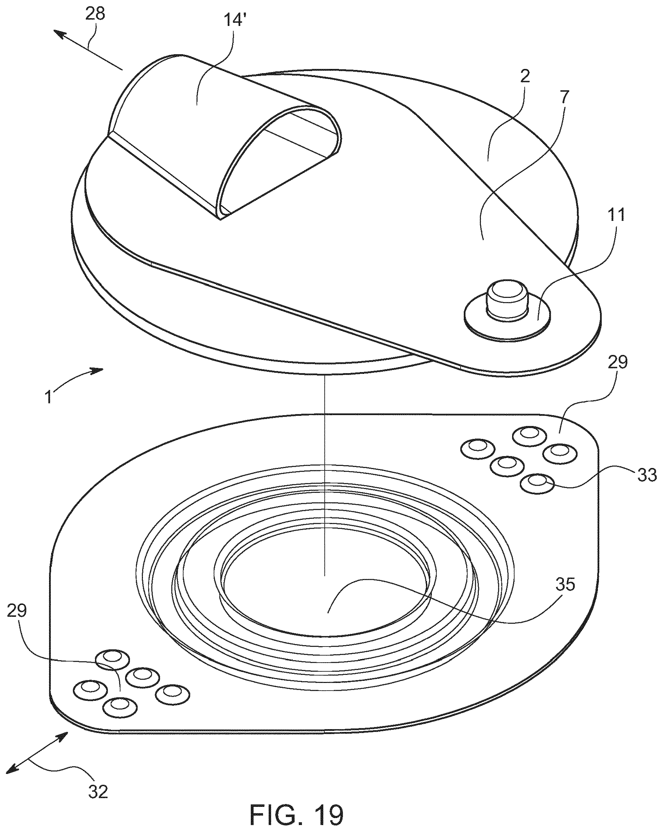

According to the embodiment of Fig. 19, the finger grip 14' defines an insert direction 28 for the insertion of a fingertip into the finger grip, and second grip flaps 29 (corresponding to a second embodiment of the protective liner 12) protrudes from the edge 30 of the disc 2 in a direction 32 at right angles to the insert direction 28 (and therefore in a transverse direction of the insert direction 28). Thereby, the application of the medical electrode 1 by means of a single hand may be further facilitated in that the grip flap 29 protruding in a transverse direction of the insert direction 28 may easily be reached by means of one or more other fingers than the finger used to hold the electrode by being inserted into the finger grip.

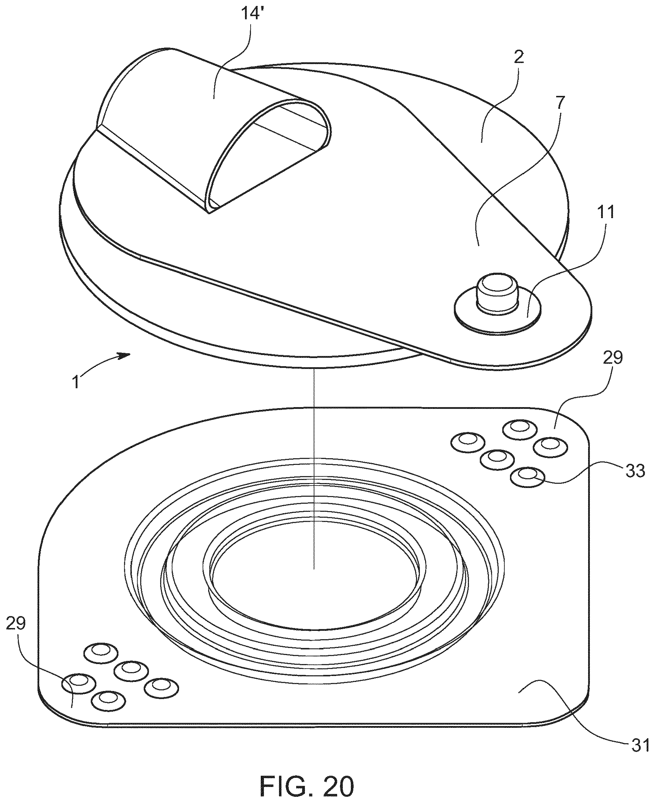

According to the embodiment of Fig. 20, the finger grip 14' defines an insert direction 28 for the insertion of a fingertip into the finger grip, and third grip flaps 29 (corresponding to a third embodiment of the protective liner 12) protrudes from the edge 30 of the disc 2 in a direction 32 at right angles to the insert direction 28 (and therefore in a transverse direction of the insert direction 28). This embodiment corresponds to the embodiment of Fig. 19 apart from the fact that the protective liner 12 is preferably also provided with a protective flap 31 arranged below the electric connector 11 as according to the embodiment illustrated in Figs. 17 and 18 in order to protect the electric connector 11 and thereby also the extension 19 of transducer strip 15. The configuration of Fig. 20 may be preferred with electrodes of large diameter, e.g. > or = 6-7 cm in diameter, to allow for the clinicians, including a clinician with small hands, to reach the grip flap 29 from one of the "sides" of the electrode to perform the one-hand removal of the protective liner 1. Also, if the liner has two grip flaps 29 one extending to each side as illustrated on Figs. 19 to 20, then the protective liner 12 can be removed using a single hand irrespective of whether the clinician is using the right hand or left hand.

According to the embodiment of Fig. 21, the finger grip 14" defines an insert direction 28 for the insertion of a fingertip into the finger grip, and fourth grip flaps 29 (corresponding to a fourth embodiment of the protective liner 12) protrudes from the edge 30 of the disc 2 in the insert direction 28 and also in a direction 32 at right angles to the insert direction 28 (and therefore in a transverse direction of the insert direction 28). As seen, one grip flap 29 protrudes in the insert direction 28 and two opposed grip flaps protrude in a direction 32 at right angles to the insert direction 28. This embodiment of the protective liner 12 is preferably also provided with a protective flap 31 arranged below the electric connector 11 as according to the embodiment illustrated in Figs. 17 and 18.

According to the embodiment of Fig. 22, the finger grip 14‴ defines an insert direction 28 for the insertion of a fingertip into the finger grip, and a fifth grip flap 29 (corresponding to a fifth embodiment of the protective liner 12) protrudes from the edge 30 of the disc 2 in the insert direction 28 and also in a transverse direction of the insert direction 28. As seen, the fifth grip flap 29 extends over a larger part of the edge 30 of the disc 2 than according to the other illustrated embodiments in order to protrude in both the insert direction 28 and directions being transverse to the insert direction 28. This embodiment of the protective liner 12 is preferably also provided with a protective flap 31 arranged below the electric connector 11 as according to the embodiments illustrated in Figs. 17, 18, 20 and 21. Preferably, the fifth grip flap 29 extends over a part of the circumferential edge 30 of the disc 2 covering at least a total angle extending from a first side of the insert direction 28 to a second side of the insert direction of 80 degrees, more preferred at least a total angle of 100 degrees, even more preferred at least a total angle of 130 degrees, and most preferred at least a total angle of 180 degrees. The fifth grip flap 29 may be arranged symmetrically in relation to the insert direction 28, but it may also be arranged in an asymmetric way in relation to the insert direction 28.

According to the embodiments of Figs. 17 to 22, the at least one grip flap 29 has an uneven surface in that it is provided with a number of surface protrusions in the form of knobs 33. Any other suitable surface protrusion(s) may be used. Thereby, the removal of the protective liner 12 from the medical electrode 1 may further be facilitated in that an even more secure grip may be provided for the finger or fingers used to press on the at least one grip flap 29. The knobs 33 may also serve as indications for where to place the fingers for removal of the protective liner 12. Furthermore, there may be provided a rough surface of the material of the at least one grip flap 29. This may also provide a more secure grip.

In an embodiment, the protective liner 12 protrudes from the edge 30 of the disc along a circumference of the disc by at least a minimum protrusion distance 34, the at least one grip flap 29 of the protective liner 12 protrudes from the edge 30 of the disc by a distance of at least 5 times, and preferably at least 8 times, the minimum protrusion distance. Thereby, as explained above, a suitable area for the at least one grip flap 29 of the protective liner 12 may be provided without using much material for the protective liner along the entire circumference of the disc. The minimum protrusion distance 34 could typically be about 2 to 5 millimetres.

In the illustrated embodiments of the medical electrode 1 according to the present disclosure, the electric connector 11 is offset in relation to a central part of the disc 2 of the medical electrode 1, and the electric connector 11, the central part of the electrode and the finger grip 14', 14", 14'" are aligned along a centre line of the medical electrode 1 extending in the insert direction 28 defined by the finger grip. However, according to the present disclosure, the illustrated finger grip 14', 14", 14'" and the at least one grip flap 29 is equally applicable to electrodes having a centred electric connector.

In the illustrated embodiments of the medical electrode 1 according to the present disclosure, an aperture 5 extends at a central part of the disc 2 from the top side 3 to the bottom side 4 and thereby forms the contact medium chamber 6 in which the contact medium 18 is arranged, and the finger grip 14', 14", 14‴ is arranged at the top side of the surrounding part of the disc 2, as particularly well seen in Fig. 23. Thereby, as explained above, the position of the finger grip 14', 14", 14‴ may induce a user to press the medical electrode against the patient's skin at the surrounding part of the disc rather than at the central part of the disc on the contact medium chamber and thereby avoid that contact medium, such as gel, may splash out from the contact medium chamber.

The finger grip 14', 14", 14'" may be detachably arranged on the medical electrode for instance by means of break lines (not shown). This may be advantageous, for instance if the patient is wearing clothes after having the electrode applied to the skin. Thereby, any inconvenience for the patient may be avoided by removal of the finger grip after placement of the electrode on the skin.

The finger grip 14', 14", 14‴ may be caught by the clothes (or bed linen), e.g. by folds, seams, edges, buttons, buttonholes, zippers or the like parts of the clothes, if not removed. This may cause a tearing effect ( e.g. when the patient is moving or walking etc.). This tearing effect may be uncomfortable to the patient. Furthermore, this tearing effect could potentially also cause mechanically induced artifacts in the bioelectrical signals, e.g. ECG signals, detected by the electrode(s). Therefore, by removing the finger grip 14', 14", 14‴ after attaching the electrode to the patient could reduce or even eliminate the risk of signal artifacts caused by mechanical impact of the clothing on the finger grip.

It is noted that whereas certain combinations of three different finger grips 14', 14", 14" and five different protective liners 12 have been illustrated in the figures, any combination of these is possible. Furthermore, according to the present disclosure, many other embodiments of the finger grip 14', 14", 14" and the protective liner 12 having at least one grip flap 29 are possible, as well as any suitable combination of these is possible.

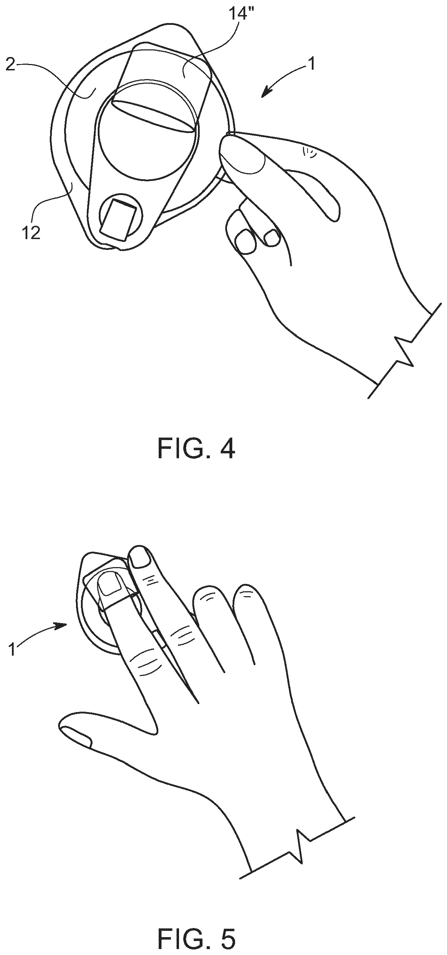

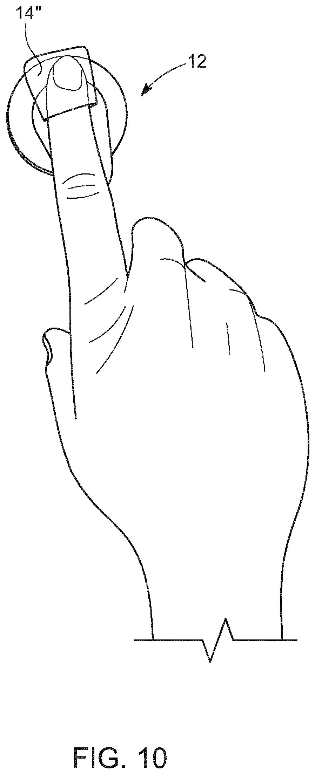

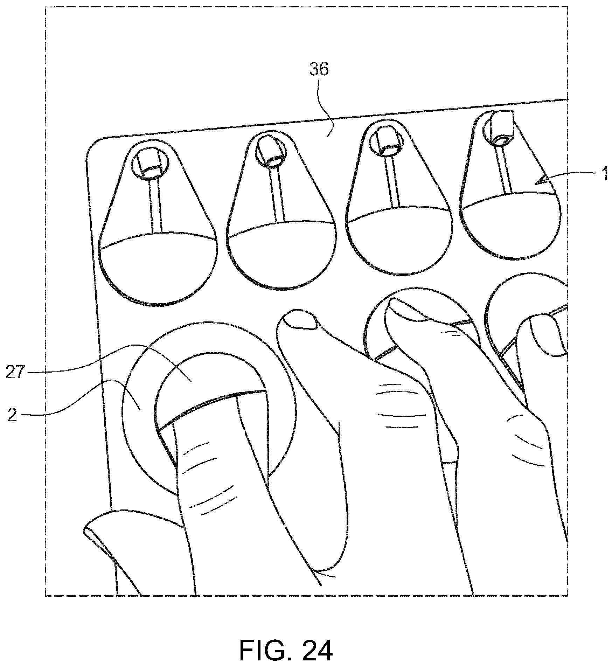

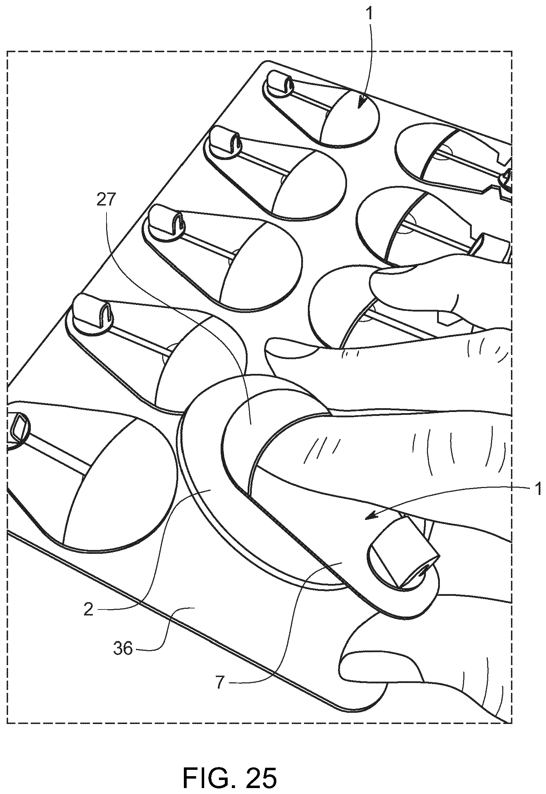

Figs. 4 to 10 illustrate the process of placement of a medical electrode 1 according to the present disclosure on the skin of a patient. The medical electrode 1 used in these figures is provided with a second finger grip 14" as illustrated for instance in Fig. 21 and a first embodiment of the protective liner 12 as illustrated in Figs. 17 and 18. The finger grip 14" allows the clinician to apply the electrode to the index finger as seen in Figs. 4 and 5 and use the thumb to remove the protective liner 12 as seen in Figs. 6 to 10. The clinician can then use the finger grip to apply the electrode 1 to the patient without touching the adhesive layer or the electrolyte well as seen in Fig. 10 which are clean areas, while identifying the correct application spot with the other hand. In fact, any combination of the finger grip with a protective liner may use a similar single handed detachment of the protective liner. Also, any type of finger grip as disclosed herein can be combined with a large sheet-like protective liner 12 carrying a plurality of medical electrodes (see e.g. Figs. 1 to 2) and can be removed from this sheet-like protective liner as described above. A further embodiment of a large sheet-like protective liner 36 carrying a plurality of medical electrodes is illustrated during application in Figs. 24 and 25.

The disc 2 does not need to be a foam disc as discussed above in relation to embodiment shown in the figures. The disc can be formed from any material used as backings in biomedical surface electrodes. Examples of suitable discs materials for disc 2 are polymeric foils or tapes or laminates, e.g. comprising one or more of the polymers listed above for the disc 2. Alternatively, the disc may comprise an upper layer of non-woven tapes or non-woven material such as rayon, viscose or polyester or mixtures thereof. The disc can also for instance be a laminate.

The finger grip 14, 14',14",14‴, 26 and/or an optional disc foil 7 at the top surface of the disc may be welded onto the upper side of the disc 2 as described above, where materials allow, or, alternatively, glued to the surface using a hotmelt or an adhesive. An example of an electrode where a finger grip may be applied to the upper surface by hotmelt or an adhesive is e.g. Ambu's Blue sensor SU, which is described in GB2341104.

Also, the medical electrode 1 does not need to comprise a central aperture filled with a gel electrolyte as discussed above. The disc 2 may have a conductive adhesive covering the entire lower surface. A conductive adhesive is able to transfer the electrical signals from the skin-adhesive interface to the transducer due to the content of ions. A conductive adhesive is e.g. a cohesive hydrogel, which comprises ions in particular chloride (Cl⁻) ions together with silver ions( Ag+), Sodium ions (Na+) and/or potassium ions (K+) whereby the conductive adhesive also can transfer electrical signals from the skin to the transducer element 15.

In the embodiments illustrated in the figures, the finger grip 14', 14", 14‴ is arranged eccentrically in relation to a central part of the disc 2 of the medical electrode 1 or the finger grip 14', 14", 14‴ is arranged at an edge 30 of the disc 2 of the medical electrode 1. Thereby, the point where the clinician will be pulling in the electrode 1 by means of the finger grip 14', 14", 14‴ may be close to the edge 30 of the disc 2. Thus, the force needed to delaminate the protective liner 12, 36 from the adhesive layer 16 on the bottom side 4 of the disc 2 may be minimized and thereby the removal of the protective liner 12, 36 may be facilitated.

### List of reference numbers

- P₁, P₂, P₃, P₄: positions of application of medical electrode
- 1: medical electrode
- 2: disc
- 3: top side of disc
- 4: bottom side of disc
- 5: aperture forming contact medium chamber
- 6: contact medium chamber
- 7: plastic foil
- 8: top side of plastic foil
- 9: bottom side of plastic foil
- 10: weld line surrounding aperture
- 11: electric connector mounted on plastic foil
- 12: protective liner
- 13: first snap lock part
- 14': first finger grip
- 14": second finger grip
- 14‴: third finger grip
- 15: transducer strip
- 16: layer of pressure-sensitive adhesive
- 17: sponge
- 18: contact medium
- 19: extension of transducer strip
- 20: finger insert opening
- 21: T-formed extension of plastic foil
- 22: first part of T-formed extension
- 23: traverse part of T-formed extension
- 24: first end of traverse part
- 25: second end of traverse part
- 26: pouch
- 27: separate piece of plastic foil
- 28: insert direction for insertion of fingertip into finger grip
- 29: grip flap
- 30: edge of disc
- 31: protective flap
- 32: direction at right angles to insert direction
- 33: knobs
- 34: minimum protrusion distance
- 35: central depression of protective liner
- 36: sheet-formed protective liner for several medical electrodes

## Claims

1. A medical electrode (1) including a disc (2) having a top side (3) and a bottom side (4), an electric connector (11) being adapted to be electrically connected to monitoring equipment, a contact medium being arranged at the bottom side (4) of the disc (2) and being electrically connected to the electric connector (11), a layer of pressure-sensitive adhesive (16) being applied to at least a part of the bottom side (4) of the disc (2), and a protective liner (12) being removably adhered to the bottom side (4) of the disc (2) by means of the pressure-sensitive adhesive (16), wherein a finger grip (14', 14", 14"') is arranged at the top side (3) of the disc (2), and wherein the finger grip forms a finger insert opening (20) into which a fingertip may be inserted and thereby hold the medical electrode (1) during handling, **characterised in that** the finger grip (14', 14", 14‴) has the form of a pouch (26) forming the finger insert opening (20).

2. A medical electrode according to claim 1, wherein said finger grip (14', 14", 14"') having the form of a pouch (26) has the form of a strap having opposed ends being attached to the medical electrode (1).

3. A medical electrode according to any one of the preceding claims, wherein said finger grip (14', 14", 14"') having the form of a pouch (26) has the form of a plastic loop that is attached to an upper surface of the medical electrode (1).

4. A medical electrode according to any one of the preceding claims, wherein the finger grip (14', 14", 14"') is tapered in such a way that an internal cross-sectional area is reduced gradually with increasing distance from the finger insert opening (20).

5. A medical electrode according to any one of the preceding claims, wherein the protective liner (12) is a separate liner for said medical electrode (1), and wherein the protective liner (12) is provided with at least one grip flap (29) protruding from an edge (30) of the disc (2).

6. A medical electrode according to claim 5, wherein the finger grip (14', 14", 14"') defines an insert direction (28) for the insertion of a fingertip into the finger grip (14', 14", 14"'), and wherein the at least one grip flap (29) protrudes in the insert direction (28) or a transverse direction thereof.

7. A medical electrode according to claim 5 or 6, wherein the at least one grip flap (29) has an uneven surface, possibly provided with a number of surface protrusions, such as knobs (33).

8. A medical electrode according to any one of the claims 5 to 7, wherein the protective liner (12) protrudes from the edge (30) of the disc (2) along a circumference of the disc by at least a minimum protrusion distance (34), wherein the at least one grip flap (14', 14", 14"') of the protective liner (12) protrudes from the edge (30) of the disc (2) by a distance of at least 5 times, and preferably at least 8 times, the minimum protrusion distance (34).

9. A medical electrode according to any one of the claims 6 to 8, wherein the electric connector (11) is offset in relation to a central part of the disc (2) of the medical electrode (1), and wherein the electric connector (11), the central part of the electrode (1) and the finger grip (14', 14", 14‴) are aligned along a centre line of the medical electrode (1) extending in the insert direction (28) defined by the finger grip (14', 14", 14‴).

10. A medical electrode according to any one of the preceding claims, wherein an aperture (5) extends at a central part of the disc (2) from the top side (3) to the bottom side (4) and thereby forms a contact medium chamber (6) in which the contact medium is arranged, and wherein the finger grip (14', 14", 14"') is arranged at the top side (3) of the surrounding part of the disc (2).

11. A medical electrode according to claim 10, wherein a plastic foil (7) having a top side (8) and a bottom side (9) is arranged on the top side (3) of the disc (2), thereby covering the aperture (5) and at least a part of the surrounding disc, the plastic foil (7) being welded to the disc along a weld line (10) surrounding the aperture (5), and wherein the finger grip (14', 14", 14"') is formed as a part of the plastic foil (7) or is attached to the plastic foil.

12. A medical electrode according to any one of the preceding claims, wherein the finger grip (14', 14", 14"') is detachably arranged on the medical electrode (1).

13. A medical electrode according to any one of the preceding claims, wherein the finger grip (14', 14", 14"') is attached to the medical electrode (1) at a distance from the electric connector (11).

14. A medical electrode according to any one of the preceding claims, wherein the finger grip (14', 14", 14"') is arranged eccentrically in relation to a central part of the disc (2) of the medical electrode (1).

15. A medical electrode according to any one of the preceding claims, wherein the finger grip (14', 14", 14"') is arranged at an edge (30) of the disc (2) of the medical electrode (1).

## Patentansprüche

1. Medizinische Elektrode (1), beinhaltend eine Scheibe (2), die eine Oberseite (3) und eine Unterseite (4) aufweist, einen elektrischen Verbinder (11), der angepasst ist, um elektrisch mit einer Überwachungsausrüstung verbunden zu werden, ein Kontaktmedium, das an der Unterseite (4) der Scheibe (2) angeordnet ist und elektrisch mit dem elektrischen Verbinder (11) verbunden ist, eine Schicht aus druckempfindlichem Klebstoff (16), die auf mindestens einen Teil der Unterseite (4) der Scheibe (2) aufgetragen ist, und eine Schutzauskleidung (12), die mittels des druckempfindlichen Klebstoffs (16) entfernbar an der Unterseite (4) der Scheibe (2) angehaftet ist, wobei ein Fingergriff (14', 14", 14"') an der Oberseite (3) der Scheibe (2) angeordnet ist, und wobei der Fingergriff eine Fingereinführöffnung (20) bildet, in die eine Fingerspitze eingeführt werden kann und dadurch die medizinische Elektrode (1) bei Handhabung gehalten werden kann, **dadurch gekennzeichnet, dass** der Fingergriff (14', 14", 14‴) die Form eines Beutels (26) aufweist, der die Fingereinführöffnung (20) bildet.

2. Medizinische Elektrode nach Anspruch 1, wobei der Fingergriff (14', 14", 14‴), der die Form eines Beutels (26) aufweist, die Form eines Riemens aufweist, der gegenüberliegende Enden aufweist, die an der medizinischen Elektrode (1) befestigt sind.

3. Medizinische Elektrode nach einem der vorstehenden Ansprüche, wobei der Fingergriff (14', 14", 14‴), der die Form eines Beutels (26) aufweist, die Form einer Kunststoffschlaufe aufweist, die an einer oberen Oberfläche der medizinischen Elektrode (1) befestigt ist.

4. Medizinische Elektrode nach einem der vorstehenden Ansprüche, wobei der Fingergriff (14', 14", 14‴) verjüngt ist, sodass sich eine innere Querschnittsfläche mit zunehmender Entfernung von der Fingereinführöffnung (20) allmählich verringert.

5. Medizinische Elektrode nach einem der vorstehenden Ansprüche, wobei die Schutzauskleidung (12) eine separate Auskleidung für die medizinische Elektrode (1) ist, und wobei die Schutzauskleidung (12) mit mindestens einer Grifflasche (29) bereitgestellt ist, die von einer Kante (30) der Scheibe (2) hervorsteht.

6. Medizinische Elektrode nach Anspruch 5, wobei der Fingergriff (14', 14", 14‴) eine Einführrichtung (28) zum Einführen einer Fingerspitze in den Fingergriff (14', 14", 14‴) definiert, und wobei die mindestens eine Grifflasche (29) in der Einführrichtung (28) oder einer Querrichtung davon hervorsteht.

7. Medizinische Elektrode nach Anspruch 5 oder 6, wobei die mindestens eine Grifflasche (29) eine unebene Oberfläche aufweist, die möglicherweise mit einer Anzahl von Oberflächenvorsprüngen, wie beispielsweise Noppen (33), bereitgestellt ist.

8. Medizinische Elektrode nach einem der Ansprüche 5 bis 7, wobei die Schutzauskleidung (12) von der Kante (30) der Scheibe (2) entlang eines Umfangs der Scheibe um mindestens eine Mindestvorsprungsdistanz (34) hervorsteht, wobei die mindestens eine Grifflasche (14', 14", 14‴) der Schutzauskleidung (12) von der Kante (30) der Scheibe (2) um eine Distanz von mindestens dem 5-fachen und vorzugsweise mindestens dem 8-fachen der Mindestvorsprungsdistanz (34) hervorsteht.

9. Medizinische Elektrode nach einem der Ansprüche 6 bis 8, wobei der elektrische Verbinder (11) im Verhältnis zu einem Mittelteil der Scheibe (2) der medizinischen Elektrode (1) versetzt ist, und wobei der elektrische Verbinder (11), der Mittelteil der Elektrode (1) und der Fingergriff (14', 14", 14‴) entlang einer Mittellinie der medizinischen Elektrode (1) ausgerichtet sind, die sich in der durch den Fingergriff (14', 14", 14‴) definierten Einführrichtung (28) erstreckt.

10. Medizinische Elektrode nach einem der vorstehenden Ansprüche, wobei sich in einem Mittelteil der Scheibe (2) eine Öffnung (5) von der Oberseite (3) zur Unterseite (4) erstreckt und dadurch eine Kontaktmedienkammer (6) bildet, in der das Kontaktmedium angeordnet ist, und wobei der Fingergriff (14', 14", 14‴) an der Oberseite (3) des umgebenden Teils der Scheibe (2) angeordnet ist.

11. Medizinische Elektrode nach Anspruch 10, wobei eine Kunststofffolie (7), die eine Oberseite (8) und eine Unterseite (9) aufweist, auf der Oberseite (3) der Scheibe (2) angeordnet ist und dadurch die Öffnung (5) und mindestens einen Teil der umgebenden Scheibe bedeckt, wobei die Kunststofffolie (7) entlang einer die Öffnung (5) umgebenden Schweißlinie (10) an die Scheibe geschweißt ist, und wobei der Fingergriff (14', 14", 14‴) als Teil der Kunststofffolie (7) gebildet ist oder an der Kunststofffolie befestigt ist.

12. Medizinische Elektrode nach einem der vorstehenden Ansprüche, wobei der Fingergriff (14', 14", 14‴) abnehmbar an der medizinischen Elektrode (1) angeordnet ist.

13. Medizinische Elektrode nach einem der vorstehenden Ansprüche, wobei der Fingergriff (14', 14", 14‴) in einem Abstand vom elektrischen Verbinder (11) an der medizinischen Elektrode (1) befestigt ist.

14. Medizinische Elektrode nach einem der vorstehenden Ansprüche, wobei der Fingergriff (14', 14", 14‴) exzentrisch im Verhältnis zu einem Mittelteil der Scheibe (2) der medizinischen Elektrode (1) angeordnet ist.

15. Medizinische Elektrode nach einem der vorstehenden Ansprüche, wobei der Fingergriff (14', 14", 14‴) an einer Kante (30) der Scheibe (2) der medizinischen Elektrode (1) angeordnet ist.

## Revendications

1. Électrode médicale (1) incluant un disque (2) présentant une face supérieure (3) et une face inférieure (4), un connecteur électrique (11) étant adapté pour être raccordé électriquement à un équipement de surveillance, un support de contact étant agencé au niveau de la face inférieure (4) du disque (2) et étant raccordé électriquement au connecteur électrique (11), une couche d'adhésif sensible à la pression (16) étant appliquée sur au moins une partie de la face inférieure (4) du disque (2), et une doublure de protection (12) étant collée de manière amovible sur la face inférieure (4) du disque (2) au moyen de l'adhésif sensible à la pression (16), dans laquelle une prise pour les doigts (14', 14", 14"') est agencée sur la face supérieure (3) du disque (2), et dans laquelle la prise pour les doigts forme une ouverture d'insertion de doigt (20) dans laquelle un bout de doigt peut être inséré et, de ce fait, maintenir l'électrode médicale (1) pendant une manipulation, **caractérisée en ce que** la prise pour les doigts (14', 14", 14‴) présente la forme d'une pochette (26) formant l'ouverture d'insertion de doigt (20).

2. Électrode médicale selon la revendication 1, dans laquelle ladite prise pour les doigts (14', 14", 14‴) présentant la forme d'une pochette (26) présente la forme d'une sangle dont des extrémités opposées sont attachées à l'électrode médicale (1).

3. Électrode médicale selon l'une quelconque des revendications précédentes, dans laquelle ladite prise pour les doigts (14', 14", 14‴) présentant la forme d'une pochette (26) présente la forme d'une boucle en plastique qui est attachée à une surface supérieure de l'électrode médicale (1).

4. Électrode médicale selon l'une quelconque des revendications précédentes, dans laquelle la prise pour les doigts (14', 14", 14‴) est effilée de telle manière qu'une aire de section transversale interne soit réduite progressivement avec l'augmentation de la distance à partir de l'ouverture d'insertion de doigt (20).

5. Électrode médicale selon l'une quelconque des revendications précédentes, dans laquelle la doublure de protection (12) est une doublure distincte pour ladite électrode médicale (1) et dans laquelle la doublure de protection (12) est pourvue d'au moins un volet de prise (29) faisant saillie à partir d'un bord (30) du disque (2).

6. Électrode médicale selon la revendication 5, dans laquelle la prise pour les doigts (14', 14", 14‴) définit une direction d'insertion (28) pour l'insertion d'un bout de doigt dans la prise pour les doigts (14', 14", 14‴), et dans laquelle le au moins un volet de prise (29) fait saillie dans la direction d'insertion (28) ou dans une direction transversale de ceux-ci.

7. Électrode médicale selon la revendication 5 ou 6, dans laquelle le au moins un volet de prise (29) présente une surface irrégulière, éventuellement pourvue d'un certain nombre de saillies de surface, telles que des boutons (33).

8. Électrode médicale selon l'une quelconque des revendications 5 à 7, dans laquelle la doublure de protection (12) fait saillie depuis le bord (30) du disque (2) le long d'une circonférence du disque d'au moins une distance de saillie minimale (34), dans laquelle le au moins un volet de prise (14', 14", 14‴) de la doublure de protection (12) fait saillie depuis le bord (30) du disque (2) d'une distance d'au moins 5 fois, et, de préférence, d'au moins 8 fois, la distance de saillie minimale (34).

9. Électrode médicale selon l'une quelconque des revendications 6 à 8, dans laquelle le connecteur électrique (11) est décalé par rapport à une partie centrale du disque (2) de l'électrode médicale (1) et dans laquelle le connecteur électrique (11), la partie centrale de l'électrode (1) et la prise pour les doigts (14', 14", 14‴) sont alignés le long d'une ligne centrale de l'électrode médicale (1) s'étendant dans la direction d'insertion (28) définie par la prise pour les doigts (14', 14", 14‴).

10. Électrode médicale selon l'une quelconque des revendications précédentes, dans laquelle un orifice (5) s'étend au niveau d'une partie centrale du disque (2) depuis la face supérieure (3) jusqu'à la face inférieure (4) et, de ce fait, forme une chambre de support de contact (6) dans laquelle le support de contact est agencé, et dans laquelle la prise pour les doigts (14', 14", 14‴) est agencée au niveau de la face supérieure (3) de la partie environnante du disque (2).

11. Électrode médicale selon la revendication 10, dans laquelle une feuille de plastique (7) présentant une face supérieure (8) et une face inférieure (9) est agencée sur la face supérieure (3) du disque (2), recouvrant, de ce fait, l'orifice (5) et au moins une partie du disque environnant, la feuille de plastique (7) étant soudée au disque le long d'une ligne de soudure (10) entourant l'orifice (5), et dans laquelle la prise pour les doigts (14', 14", 14‴) est formée comme une partie de la feuille de plastique (7) ou est fixée à la feuille de plastique.

12. Électrode médicale selon l'une quelconque des revendications précédentes, dans laquelle la prise pour les doigts (14', 14", 14‴) est agencée de manière amovible sur l'électrode médicale (1).

13. Électrode médicale selon l'une quelconque des revendications précédentes, dans laquelle la prise pour les doigts (14', 14", 14‴) est attachée à l'électrode médicale (1) à une certaine distance du connecteur électrique (11).

14. Électrode médicale selon l'une quelconque des revendications précédentes, dans laquelle la prise pour les doigts (14', 14", 14‴) est agencée de manière excentrique par rapport à une partie centrale du disque (2) de l'électrode médicale (1).

15. Électrode médicale selon l'une quelconque des revendications précédentes, dans laquelle la prise pour les doigts (14', 14", 14‴) est agencée au niveau d'un bord (30) du disque (2) de l'électrode médicale (1).
